# EUROPEAN PATENT SPECIFICATION

(11) **EP 1 090 929 B1**
(45) Date of publication and mention of the grant of the patent: **03.08.2005**
(21) Application number: 00308716.0
(22) Date of filing: 04.10.2000
(51) Int. Cl.: C08B 30/10, A61L 15/00, C08B 37/14

(54) **Isolation and utilisation of pentosans from by-products of the starch industry**
Isolierung und Verwendung von Pentosane aus Nebenprodukten der Stärke-Industrie
Isolation et utilisation de pentosanes à partir de sous-produits de l'industrie d'amidon

(30) Priority: 08.10.1999 GB 9923784
(43) Date of publication of application: 11.04.2001
(73) Proprietor: CERESTAR HOLDING B.V., 4551 LA Sas Van Gent (NL)
(72) Inventor: De Cock, Nicole S.J., 2220 Heist-op-den-Berg (BE); De Sadeleer, Jos Willy Ghislain Corneel, 3220 Holsbeek (BE)
(74) Representative: Wilkinson, Stephen John

(56) References cited:
- WO-A-98/27117
- DE-A- 1 924 900
- DE-A- 3 405 208
- US-A- 2 218 567
- US-A- 5 430 142

## Description

### Technical field

The present invention relates to the modification of pentosans from cereals. Specifically, the pentosans are heat- and alkali treated. Optionally, the pentosans are crosslinked using a cross-linking agent. After neutralisation the pentosan derivatives are, depending on the desired application, washed and dried. The pentosan derivatives show a high water binding capacity and are used for this capacity as component of super-absorbers in tissue paper, hygienic products, cat box filler and as binder in agricultural soil covers or in solid-state fermentation media.

### Background of the invention

Pentosans are polysaccharides predominantly consisting of arabinose substituted xylans. They can be found in large quantities in the cell walls of grain kernels, where they participate in maintaining the structural integrity of the cell wall. Pentosans are particularly found in wheat, where they typically form about 2 % of the milled flour. Wheat is used in large quantities in the starch industry. A typical process for obtaining starch from wheat comprises the following steps.
Wheat is grinded to form flour. The flour is mixed with water in a mixer and a dough is formed. The dough is washed and diluted giving a dough solution containing suspended starch and particulate gluten. Gluten and starch are separately recovered from the dough solution. The remaining solution and the wash waters contain pentosan. Concentration of these streams on centrifuges or screens can yield streams containing more than 10% pentosan. A typical composition has 10-45% pentosan, 5-20% protein and 20-70% starch. An example is 'liquid feed' (Cerestar wheat starch C) sold in the pig feed industry with a composition of about 7% protein, 13% pentosan and 60% starch.

The pentosans derived from the cell wall material of the inner endosperm are unusual in that they are water soluble to the extent of 20-25%(w/w). Highly viscous solutions are formed at concentrations as low as 1 % (w/w).
Wheat pentosans are generally used to improve bread quality. The pentosans have a high water binding capacity and as a consequence bread becomes less dry and stays fresh for a longer time, the so-called anti-staling effect. Other applications for wheat pentosans have not been extensively explored to date, and this is mainly due to the fact that are difficult to dry and give a impair a high viscosity to solutions at low dry substance content.

Waterbinding materials/absorbers are primarily used in the field of hygiene products and in building and construction. In hygiene products absorbers are found in diapers, napkins, tissues and medical products. In the building industry they are added to cement and concrete to control for example dust formation and to prevent fire. Other applications are in such diverse areas as waterbeds, optical cable insulation and sanitary products for cats.

Essential characteristics of absorbers are a high water binding capacity, which should be maintained when pressure is applied. Furthermore, the water binding capacity is preferably not influenced by the presence of salts. Absorbers, which are currently used on a large-scale are mainly organic polymers. The increasing use of this type of products gives rise to a growing environmental pressure. It would be desirable to produce absorbers based on biological and biodegradable products.

Biological and biodegradable absorber can be used as binder in typical agricultural products such as Biotop™, Terra Star (De Boomkwekerij 19,1999) which prevent weed growing by the formation of a degradable soil covering layer. These layers compete with plastic foils and herbicides.

Media for solid-state fermentation need to bind large quantities of water. Addition of an absorber could increase the water holding capacity of the medium. An absorber for this application must preferably be biological and biodegradable.

A process for chemically modifying polysaccharides, including pentosans, is described in DE-A-1924900. According to this, pentosans may be crosslinked to produce a derivative which has increased viscosity. DE-A-3405208 discloses a process for preparing a water binding agent based on hemicellulose which process includes alkali treatment, aliphatic alcohol precipitation and ultrafiltration. WO-A-9827117 describes a superabsorbent polysaccharide derivative obtained by the oxidation of polysaccharide and subsequent treatment with a crosslinking agent.

### Summary of the invention

The invention discloses the treatment of the process streams containing pentosans in such a way that the pentosans become more water absorbing. Accordingly, the invention provides a process for treating pentosans comprising the following steps:
a) heating a pentosan-containing fraction to 40-120°C, preferably above 60°C,
b) treating a pentosan fraction with alkali,
c) adding a reactive phosphate reagent wherein the ratio of pentosan to other components is between 27.6/8/8 and 27.6/0.25/0 pentosan/alkali/phosphate,
d) neutralising the product with an acid, and
e) collecting the treated pentosan.

The pentosan fraction, typically containing at least 10% pentosan (d.b.), is heated and mixed with alkali, then a reactive phosphate reagent is added and the mixture is subsequently neutralised and the pentosans are collected.
The invention discloses also the water extraction of the formed salt, the binder precipitation in ethanol and/or drying of the binder.
The present invention therefore also discloses new pentosan compositions derived from cereals, preferably wheat.
The present invention further discloses a new pentosan composition obtainable by the process, specifically new wheat pentosan derivatives, characterized in that the pentosans are capable of binding at least 20g water/g of pentosan, preferably at least 100g water/g pentosan. When salt is present in the water this value is somewhat lower. The pentosan derivatives are obtained by chemical modification.
Finally, the invention discloses the use of the new wheat pentosan compositions as binders, specifically in covering materials for soil or in solid-state fermentation media, and as super absorbers or components thereof tissue paper, hygiene products and cat box filler.

### Brief description of the figures

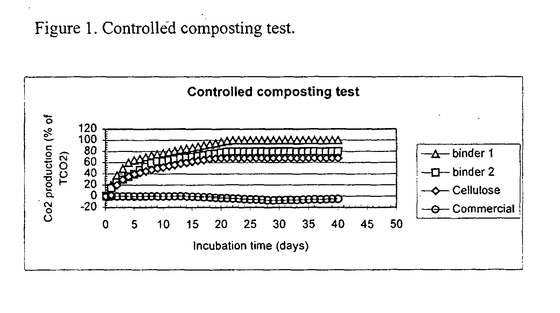

Figure 1 shows the results of a biodegradability test comparing two different pentosan binders with cellulose and a commercial binder based on poly-acrylates.

### Detailed description of the invention

The present invention discloses the treatment of a pentosan containing stream to make the pentosans more water absorbing. In principle all process streams containing a sufficient amount of pentosan can be applied as a starting material for the present invention. A typical composition of a pentosan stream contains from 10-45% pentosan, from 5-20% protein and from 20-70% starch on a dry weight basis. An example of such a stream is 'liquid feed' (Cerestar Wheat Starch C) sold in the pig feed industry with a composition of about 7% protein, 13% pentosan and 60% starch. The pentosan stream or fraction containing at least 10% pentosan (d.b.) is heated to 40-120°C, preferably above 60°C, mixed with alkali, the mixture is neutralised after 1 min to 10 hours, preferably after 30 minutes and the pentosans are collected. The invention discloses also the reaction of a reactive phosphate reagent with the alkaline pentosan stream.
The invention further discloses the water extraction of the formed salt, and the subsequent precipitation of the water binding pentosan fraction in ethanol and/or drying. Other lower alcohols such as propanol, isopropanol and butanol are also usable in the present invention.
Binders have been prepared containing different ratios of pentosans/alkali/phosphate ratios differ from 27.6/8/8 to 27.6/0.25/0
The alkali added was in the form of alkali metal hydroxides, preferred among these are sodium- and calciumhydroxide.
The phosphates, which are used as active reagents may be chosen from sodium tri-meta phosphate (STMP), sodium tri-penta phosphate (STPP), sodium di-hydrogen phosphate (SDHP) and di-sodium pyrophosphate (DSPP).
The neutralising acids are chosen from organic or inorganic acids, preferred are hydrochloric acid and phosphoric acid.

The pentosans useful in the present invention are derived from cereals. Although the preferred source is wheat it is also possible to use corn, barley or triticale as source for the pentosans.

The present invention therefore also discloses new pentosan composition specifically new wheat pentosan derivatives characterized in that they are obtained according to the process of any one of claims 1 to 9 and that they are capable of binding at least 20g water/g of pentosan, preferably at least 100g water/ g pentosan. Values of up to 250 g water/ g pentosan have been measured. When salt is present in the water this value is somewhat lower but it is still ate least about 20g physiological salt containing water/g pentosan. The pentosan derivatives are obtained by chemical modification.

The pentosan derivatives are used in certain binder compositions, to which end they are further mixed with other ingredients depending of their application. When used for example as a cat box filler the pentosan was mixed with normal vegetable biodegradable cat box filler; the pentosans are added in the desired amount which goes up to 10 %(d.s) preferably, 25 or 30 % (d.s) is added. The so-obtained cat box filler shows considerable increase in water retention over the normal filler. Increase in water retention values of up to 40 % have been achieved. In addition the combined product is biodegradable.
When used for example to cover agricultural soil the pentosans of the present invention are mixed with straw, palm oil and phosphoric acid, a composition as used in European patent EP 0782 602 B1 but replacing the expensive starch by the pentosans derivatives of the present invention.

Finally, the invention discloses the use of the pentosan compositions as super absorbers or as ingredients thereof in hygienic products.

The present invention is disclosed in more detail in the following examples.

### Experimental

Abbreviations used in the examples.
STMP = sodium tri-meta phosphate
STPP = sodium tri-penta phosphate
SDHP = soduim di-hydrogen phosphate
DSPP = di-sodium pyrophosphate

### Example 1

A binder was prepared following the dry weight ratio recipe: pentosan fraction/NaOH/STMP = 27.6/8/8 and neutralised with HCl. The formed salts were removed by water extraction. The remaining binder was precipitated in ethanol and air-dried. The binder had a water holding capacity of 250g/g in water and 20g/g in physiological salt solution. The water binding capacity of the binder is 7g/g in water and 7g/g in physiological salt solution both under 2kPa pressure.

### Example 2

Two binders were prepared according to the following dry weight ratio recipes and neutralised with HCl:
- binder 1: pentosan fraction/bran/NaOH/STMP = 19.6/8/8/8
- binder 2: pentosan fraction/NaOH/STMP = 27.6/8/8

The formed salts were removed by water extraction and the remaining binders were precipitated in ethanol and air-dried.
The two binders were brought in a controlled composting test together with a commercial absorber based on poly-acrylates and a cellulose sample as reference. The biodegradability of the binders was good, while the commercial product showed at the end of the test no sign of degradation. See figure 1.

### Example 3

A binder was prepared following the dry weight ratio recipe: pentosan fraction/NaOH/STMP = 27.6/8/8 and neutralised with HCl. The formed salts were removed by water extraction. The remaining binder was precipitated in ethanol and air-dried.
A vegetable, biological degradable cat's box filler was mixed with the binder in a weight ratio of 10:0; 10:0.5; 10:1; 10:2.5. The mixture had a water holding capacity in water of 100%, 144%, 227%, and 296% for increasing binder ratio. The water holding capacity in physiological salt solution is 100%, 110%, 134% and 154% for increasing binder ratio.

### Example 4

A binder was prepared according to example 3. A lime based, natural cat box filler was mixed with the binder in a weight ratio of 10:0; 10:0.5; 10:1; 10:2.5. The mixture had a water holding capacity in water of 100%, 126%, 163%, and 216% for increasing binder ratio. The water holding capacity in physiological salt solution is 100%, 131%, 163% and 275% for increasing binder ratio.

### Example 5

Different types of binders were prepared following the dry weight ratio recipe: pentosan fraction/alkali/STMP and neutralised with an acid as described in the following table. The formed salts were not removed out of the binder.
Mixtures of these binders with finely divided straw, palm oil and phosphoric acid were made in a dry weight ratio following the dry weight recipe: straw/binder/palm oil/phosphoric acid as described in the table.

| | **Binder** | | | **Mixture** |
|---|---|---|---|---|
| **NR.** | **Recipe** | **Alkali type** | **Acid type** | **Recipe** |
| 1 | 27.6/4/4 | NaOH | HCl | 58.7/29.3/10.6/1.4 |
| 2 | 27.6/4/4 | Ca(OH)2 | H3PO4 | 58.7/29.3/10.6/1.4 |
| 3 | 27.6/4/4 | Ca(OH)2 | KGA | 58.7/29.3/10.6/1.4 |
| 4 | 27.6/4/0 | NaOH | HCl | 58.7/29.3/10.6/1.4 |
| 5 | 27.6/4/0 | Ca(OH)2 | H3PO4 | 58.7/29.3/10.6/1.4 |
| 6 | 27.6/4/0 | Ca(OH)2 | KGA | 58.7/29.3/10.6/1.4 |
| 7 | 27.6/4/4 | NaOH | HCl | 67.1/19.4/12.1/1.4 |
| 8 | 27.6/4/4 | Ca(OH)2 | H3PO4 | 67.1/19.4/12.1/1.4 |
| 9 | 27.6/4/4 | Ca(OH)2 | KGA | 67.1/19.4/12.1/1.4 |
| 10 | 27.6/4/0 | NaOH | HCl | 67.1/19.4/12.1/1.4 |
| 11 | 27.6/4/0 | Ca(OH)2 | H3PO4 | 67.1/19.4/12.1/1.4 |
| 12 | 27.6/4/0 | Ca(OH)2 | KGA | 67.1/19.4/12.1/1.4 |
| 13 | 27.6/4/4 | NaOH | HCl | 50.4/39.1/9.1/1.4 |
| 14 | 27.6/4/4 | Ca(OH)2 | H3PO4 | 50.4/39.1/9.1/1.4 |
| 15 | 27.6/4/4 | Ca(OH)2 | KGA | 50.4/39.1/9.1/1.4 |
| 16 | 27.6/4/0 | NaOH | HCl | 50.4/39.1/9.1/1.4 |
| 17 | 27.6/4/0 | Ca(OH)2 | H3PO4 | 50.4/39.1/9.1/1.4 |
| 18 | 27.6/4/0 | Ca(OH)2 | KGA | 50.4/39.1/9.1/1.4 |

The mixtures were diluted with water to 10% dry matter and spread over cultivation soil. After drying, layers were formed which prevented the growth of weeds for several months. Under all weather conditions

### Example 6

Different types of binder and mixtures were prepared according to example 5 were the quantity of STMP and alkali in the binder recipe is divided respectively by a factor 2 and 4 to reduce the quantity of salts. The mixtures were diluted to 10% dry matter and spread over cultivation soil. After drying, layers were formed which prevented the growth of weeds for several months.

### Example 7

Different types of binder and mixtures were prepared according to example 5 were the quantity of alkali in the binder recipe is decreased to a level were a pH of at least 10 is reached during the reaction. The acid for neutralisation is decreased with the same level resulting in a binder with a minimal quantity of salts. The mixtures were diluted to 10% dry matter and spread over cultivation soil. After drying, layers were formed which prevented the growth of weeds for several months.

### Example 8

Different types of binders and mixtures were prepared according to examples 5,6 and 7 with replacement of the finely divided straw by wheat bran in the mixture recipe. The mixtures were diluted to 10% dry matter and spread over cultivation soil. After drying, layers were formed which prevented the growth of weeds for several months.

### Example 9

Different types of binders and mixtures were prepared according to examples 5,6 and 7 with replacement of the finely divided straw by a 50/50-weight mixture of fine divided straw and bran in the mixture recipe. The mixtures were diluted to 10% dry matter and spread over cultivation soil. After drying, layers were formed which prevented the growth of weeds for several months.

### Example 10

Different types of binders and mixtures were prepared according to examples 5,6,7,8 and 9 with replacement of a part or the total quantity of the STMP by STPP, SDHP or DSPP. The mixtures were diluted to 10% dry matter and spread over cultivation soil. After drying, layers were formed which prevented the growth of weeds for several months.

### Example 11

Different type of binders and mixtures were prepared according to examples 10. The mixtures were dried in an air dryer and homogenised in a granulator to a dry matter of >85%. The dried mixtures were dry spread over cultivation soil and wetted by water sprinklers or by rain. After drying, layers were formed which prevented the growth of weeds for several months. The dried mixtures could easily be spread by for example a dry fertiliser spreader.

### Example 12

Different type of binders and mixtures were prepared according to example 11. The dried mixtures were re-suspended to 10% dry matter. The re-suspended mixtures were spread over cultivation soil. After drying, layers were formed which prevented the growth of weeds for several months.

## Claims

1. A process for treating pentosans comprising the following steps:
a) heating a pentosan-containing fraction to 40-120°C, preferably above 60°C,
b) treating the pentosan fraction with alkali,
c) adding a reactive phosphate reagent wherein the ratio of pentosan to other components is between 27.6/8/8 and 27.6/0.25/0 pentosan/alkali/phosphate,
d) neutralising the product with an acid, and
e) collecting the treated pentosan.

2. A process according to claim 1, wherein steps a) and b) are reversed.

3. A process according to either one of claim 1 or claim 2, wherein the pentosan is derived from a cereal, preferably wheat.

4. A process according to any one of claims 1 to 3, wherein the formed salts in the neutralised product are water extracted.

5. A process according to any one of claims 1 to 4, wherein the product is precipitated with an organic solvent.

6. A process according to claim 5, wherein the organic solvent is selected from the lower alcohols, i.e. ethanol, propanol, isopropanol or butanol.

7. A process according to any one of claims 1 to 6, wherein the product is dried.

8. A process according to any one of claims 1 to 7, wherein the pentosan fraction contains at least 10% pentosan (d.b.).

9. A process according to any one of claims 1 to 8, wherein the alkali is sodium hydroxide or calcium hydroxide.

10. A pentosan derivative obtainable according to a process of any one of claims 1 to 9 and capable of binding at least 20g water/g of pentosan, preferably at least 100g water/g pentosan.

11. A cat box filler comprising a pentosan derivative according to claim 10.

12. A covering material for agricultural soil comprising a pentosan derivative according to claim 10.

13. Use of a pentosan derivative according to claim 10 as binder in tissue paper or hygienic product.

14. Use of a pentosan derivative according to claim 10 as binder in solid state fermentation medium.

## Patentansprüche

1. Verfahren zum Behandeln von Pentosanen, umfassend die nachstehenden Schritte:
a) Erhitzen einer Pentosan enthaltenden Fraktion auf 40-120°C, vorzugsweise über 60°C,
b) Behandeln der Pentosanfraktion mit Alkali,
c) Zusetzen eines reaktiven Phosphatreagenz, worin das Verhältnis von Pentosan zu anderen Komponenten zwischen 27,6/8/8 und 27,6/0,25/0 Pentosan/Alkali/Phosphat ist,
d) Neutralisieren des Produkts mit einer Säure, und
e) Sammeln des behandelten Pentosans.

2. Verfahren nach Anspruch 1, wobei Schritte a) und b) umgekehrt werden.

3. Verfahren nach einem von Anspruch 1 oder Anspruch 2, wobei das Pentosan von einem Getreide, vorzugsweise Weizen, stammt ist.

4. Verfahren nach einem der Ansprüche 1 bis 3, wobei die in dem neutralisierten Produkt gebildeten Salze mit Wasser extrahiert werden.

5. Verfahren nach einem der Ansprüche 1 bis 4, wobei das Produkt mit einem organischen Lösungsmittel ausgefällt wird.

6. Verfahren nach Anspruch 5, wobei das organische Lösungsmittel aus den Niederalkoholen, d.h. Ethanol, Propanol, Isopropanol oder Butanol, ausgewählt wird.

7. Verfahren nach einem der Ansprüche 1 bis 6, wobei das Produkt getrocknet wird.

8. Verfahren nach einem der Ansprüche 1 bis 7, wobei die Pentosanfraktion mindestens 10 % Pentosan (d.b.) enthält.

9. Verfahren nach einem der Ansprüche 1 bis 8, wobei das Alkali Natriumhydroxid oder Calciumhydroxid ist.

10. Pentosanderivat, erhältlich gemäß einem Verfahren nach einem der Ansprüche 1 bis 9 und mit der Fähigkeit, mindestens 20 g Wasser/g Pentosan, vorzugsweise mindestens 100 g Wasser/g Pentosan, zu binden.

11. Katzenstreu, umfassend ein Pentosanderivat nach Anspruch 10.

12. Bedeckungsmaterial für Agrarboden, umfassend ein Pentosanderivat nach Anspruch 10.

13. Verwendung eines Pentosanderivats nach Anspruch 10 als Bindemittel in Tissuepapier oder Hygieneprodukt.

14. Verwendung eines Pentosanderivats nach Anspruch 10 als Bindemittel in einem Festphasenfermentationsmedium.

## Revendications

1. Procédé pour le traitement des pentosanes comprenant les étapes suivantes :
a) chauffage d'une fraction contenant du pentosane à une température de 40 à 120° C, de préférence à plus de 60° C,
b) traitement de la fraction de pentosane avec de l'alcali,
c) ajout d'un réactif de phosphate réactif dans lequel le rapport du pentosane aux autres composants est compris entre 27,6/8/8 et 27,6/0,25/0 pentosane/alcali/phosphate
d) neutralisation du produit avec un acide, et
e) collecte du pentosane traité.

2. Procédé selon la revendication 1, dans lequel les étapes a) et b) sont inversées.

3. Procédé selon l'une quelconque des revendications 1 ou 2, dans lequel le pentosane est dérivé d'une céréale, de préférence du blé.

4. Procédé selon l'une quelconque des revendications 1 à 3, dans.lequel les sels formés dans le produit neutralisé sont extraits par l'eau.

5. Procédé selon l'une quelconque des revendications 1 à 4, dans lequel le produit est précipité avec un solvant organique.

6. Procédé selon la revendication 5, dans lequel le solvant organique est sélectionné parmi les alcools inférieurs, c'est-à-dire l'alcool éthylique, l'alcool propylique, l'alcool isopropylique ou l'alcool butylique.

7. Procédé selon l'une quelconque des revendications 1 à 6, dans lequel le produit est séché.

8. Procédé selon l'une quelconque des revendications 1 à 7, dans lequel la fraction de pentosane contient au moins 10 % de pentosane (base sèche).

9. Procédé selon l'une quelconque des revendications 1 à 8, dans lequel l'alcali est de l'hydroxyde de sodium ou de l'hydroxyde de calcium.

10. Dérivé de pentosane pouvant être obtenu selon un procédé selon l'une quelconque des revendications 1 à 9 et capable de lier au moins 20 g d'eau par gramme de pentosane, de préférence au moins 100 g d'eau par gramme de pentosane.

11. Matière de litière pour chat comprenant un dérivé de pentosane selon la revendication 10.

12. Matériau de couverture pour sol agricole comprenant un dérivé de pentosane selon la revendication 10.

13. Utilisation d'un dérivé de pentosane selon la revendication 10 comme liant dans du papier de soie ou un produit hygiénique.

14. Utilisation d'un dérivé de pentosane selon la revendication 10 comme liant dans un milieu de fermentation à l'état solide.
